# EUROPÄISCHE PATENTSCHRIFT

(11) **EP 1 292 263 B1**
(45) Veröffentlichungstag und Bekanntmachung des Hinweises auf die Patenterteilung: **02.11.2005**
(21) Anmeldenummer: 01938256.3
(22) Anmeldetag: 09.06.2001
(51) Int. Cl.: A61K 7/06, A61K 7/135

(54) **VITAMIN B6-DERIVATE ALS PFLEGEKOMPONENTEN IN DER OXIDATIVEN HAARBEHANDLUNG**
VITAMIN B6 DERIVATIVES AS PROTECTIVE COMPONENTS IN THE OXIDATIVE TREATMENT OF HAIR
DERIVES DE LA VITAMINE B6 EN TANT QU'AGENT DE SOIN DANS LE TRAITEMENT CAPILLAIRE OXYDANT

(30) Priorität: 20.06.2000 DE 10030313; 26.04.2001 DE 10120305
(43) Veröffentlichungstag der Anmeldung: 19.03.2003
(73) Patentinhaber: Henkel Kommanditgesellschaft auf Aktien, 40589 Düsseldorf (DE)
(72) Erfinder: KLEEN, Astrid, 40699 Erkrath (DE)
(86) Internationale Anmeldenummer: PCT/EP2001/006547
(87) Internationale Veröffentlichungsnummer: WO 2001/097755

(56) Entgegenhaltungen:
- WO-A-95/17157
- WO-A-98/51265
- DE-A- 19 613 567
- US-A- 5 681 554
- DATABASE WPI Section Ch, Week 198021 Derwent Publications Ltd., London, GB; Class D21, AN 1980-37238C XP002183448 SUN STAR HAMIKAGI: "Hair nourishing composition containing pyridoxine and pyrrolidone-carboxylic acid, or pyridoxine-pyrrolidone-carboxylic acid salt" & JP 55 049306 A (SUN STAR HAMIGAKI), 9. April 1980 (1980-04-09)
- CHEMICAL ABSTRACTS, vol. 107, no. 20, 16. November 1987 (1987-11-16) Columbus, Ohio, US; abstract no. 183330, H.KOJIMA, J.TAKENAKA: "Hair preparations containing reducing agents, sequestering agents, metallic salts, dyes and oxidizing agents" XP002183446 & JP 62 132813 A (NISHIRENJI TRADING K.K.) 1987
- CHEMICAL ABSTRACTS, vol. 107, no. 18, 2. November 1987 (1987-11-02) Columbus, Ohio, US; abstract no. 161377, S.IWAO E.A.: "Nonaerosol foam-producing cosmetics" XP002183447 & JP 62 135410 A (LION) 1987

## Beschreibung

Die Erfindung betrifft die Verwendung von Vitamin B6-Derivaten zur Verringerung der Schädigung keratinischer Fasern, insbesondere menschlicher Haare, durch oxidative Prozesse. Darüber hinaus betrifft die Erfindung Zubereitungen zur oxidativen Haarbehandlung, insbesondere zur Blondierung, die als strukturstabilisierende Komponente Vitamin B6-Derivate enthalten. Weiterhin betrifft die Erfindung ein Verfahren zur oxidativen Behandlung von Keratinfasern.

Unter Keratinfasern werden erfindungsgemäß Pelze, Wolle, Federn und insbesondere das menschliche Haar verstanden. Das menschliche Haar wird heute in vielfältiger Weise mit haarkosmetischen Zubereitungen behandelt. Dazu gehören etwa die Reinigung der Haare mit Shampoos, die Pflege und Regeneration mit Spülungen und Kuren sowie das Bleichen, Färben und Verformen der Haare mit Färbemitteln, Tönungsmitteln, Wellmitteln und Stylingpräparaten. Die oxidative Haarbehandlung ist bei der permanenten Haarfärbung mit Oxidationsfärbemitteln, bei der Haarblondierung und der dauerhaften Haarverformung unabdingbar.

Die Blondierung bzw. die Entfärbung der Haare erfolgt durch eine Oxidation der natürlichen Haarpigmente bzw. durch eine Oxidation des künstlichen Haarfarbstoffes. Die Grundlagen der Blondierverfahren sind dem Fachmann bekannt und in einschlägigen Monographien, z.B. von K. Schrader, Grundlagen und Rezepturen der Kosmetika, 2. Auflage, 1989, Dr. Alfred Hüthig Verlag, Heidelberg, oder W. Umbach (Hrg.), Kosmetik, 2. Auflage, 1995, Georg Thieme Verlag, Stuttgart, New York, zusammenfassend beschrieben. Neben dem gewünschten Effekt der Entfärbung können Blondiermittel insbesondere bei häufiger Anwendung eine Schädigung der Struktur des Haarkeratins bewirken. Besonders bei der Verwendung von sogenannten "Booster"-Komponenten, die sich in höheren Konzentrationen in den fertigen Zubereitungen lösen lassen, wie beispielsweise Ammoniumperoxodisulfat, ist die Gefahr einer verstärkten Haarschädigung gegeben. Eine zunehmende Brüchigkeit und die erschwerte Kämmbarkeit des Haars sowie eine Verschlechterung von Sitz und Fülle der Frisur sind die Folge der Haarschädigung. Darüber hinaus weist strukturgeschädigtes Haar ein stumpfes und glanzloses Aussehen auf. Diesen Problemen sollte mit strukturverbessernden Zusatzstoffen entgegengewirkt werden, z.B. im Rahmen des Blondierverfahrens, in welchem die Zusatzstoffe vorteilhafterweise ein Bestandteil des Blondiermittels selbst sind.

Aus diesen genannten Gründen besteht ein Bedarf an neuen Pflegewirkstoffen, die eine Verbesserung der erwähnten Parameter ermöglichen und mit oxidierenden Haarbehandlungsmitteln kompatibel sind.

Pyridoxin und weitere Verbindungen der Vitamin B6-Gruppe sind als Komponenten in Haartonika zur Verringerung des Nachfettens und zur Stimulierung des Haarwuchses erwähnt worden. In EP 0678293 A2 werden topische Zusammensetzungen mit einem Gehalt von Pyridoxintripropionat zur Behandlung des Haars und der Haut vorgeschlagen. In EP 001079 A1 sind kosmetische Zusammensetzungen mit antiseborrhoischer Wirkung beschrieben, die Pyridoxin-tripalmitat als Wirkstoff enthalten. Derivate des Pyridoxins, Pyridoxals oder Pyridoxamins als Wirkstoffe in oxidierenden Haarbehandlungmitteln sind dem Fachmann bisher nicht bekannt.

Überraschenderweise wurde gefunden, daß Pyridoxin-, Pyridoxal- oder Pyridoxamin-Derivate die oben genannten Anforderungen in hervorragender Weise erfüllen. Vitamin B6 sowie bestimmte Derivate bewirken auch während der oxidativen Haarbehandlung eine Verbesserung der Struktur des Haarkeratins, ohne das Aufhellergebnis oder Dauerwellergebnis zu beeinträchtigen.

Ein erster Gegenstand der Erfindung ist daher die Verwendung von Vitamin B6-Derivaten ausgewählt aus der gruppe Pyridoxin, Pyridoxal, Pyridoxamin, oder Pyridoxal-5'- phosphat, zur Verringerung der Schädigung Keratiniser Fasern, während der oxidativen Haarbehandlung.

Ein zweiter Gegenstand der Erfindung ist eine Zubereitung zur Blondierung von Keratinfasern, insbesondere dem menschlichen Haar, enthaltend mindestens ein Oxidationsmittel, dadurch gekennzeichnet, daß die Zubereitung mindestens ein Vitamin B6-Derivat, aus gewählt aus der Gruppe Pyridoxin, Pyridoxal, Pyridoxamin, oder Pyridoxal-5'-phosphat oder eines der entsprechenden physiologisch verträglichen Salze, bevorzugt in einer Menge von 0.05-2 Gew.% bezogen auf das Mittel, enthält.

Die erfindungsgemäßen Zubereitungen enthalten bevorzugt als Oxidationsmittel Wasserstoffperoxid oder eine Anlagerungsverbindung von Wasserstoffperoxid an anorganische oder organische Verbindungen, wie beispielsweise Natriumperborat, Natriumpercarbonat, Natriumpercarbamid, Polyvinylpyrrolidon, Harnstoffperoxid und Melaminperoxid.

Die Blondierwirkung kann bervorzugt durch sogenannte "Booster" gesteigert werden. Dies sind in der Regel feste Peroxoverbindungen, die keine Anlagerungsprodukte von Wasserstoffperoxid an andere Komponenten darstellen. Die Auswahl dieser Peroxoverbindungen unterliegt prinzipiell keinen Beschränkungen; übliche, dem Fachmann bekannte Peroxoverbindungen sind beispielsweise Ammoniumperoxodisulfat, Kaliumperoxodisulfat, Natriumperoxodisulfat, Ammoniumpersulfat, Kaliumpersulfat, Natriumpersulfat, Kaliumperoxodiphosphat, Percarbonate wie Magnesiumpercarbonat und Peroxide wie Bariumperoxid. Unter diesen Peroxoverbindungen, die auch in Kombination eingesetzt werden können, sind erfindungsgemäß die anorganischen Verbindungen bevorzugt. Besonders bevorzugt sind die Peroxodisulfate, insbesondere Ammoniumperoxodisulfat.

Die Peroxoverbindungen sind in den erfindungsgemäßen Blondiermitteln bevorzugt in Mengen von 1-30 Gew.-%, insbesondere in Mengen von 5-20 Gew.-%, enthalten.

Als weitere wichtige Komponente enthalten die erfindungsgemäßen Zubereitungen ein Alkalisierungsmittel, das zur Einstellung des alkalischen pH-Wertes der Anwendungsmischung dient. Erfindungsgemäß können die dem Fachmann ebenfalls für Blondiermittel bekannten, üblichen Alkalisierungsmittel wie Ammonium-, Alkalimetall-und Erdalkalimetallhydroxide, -carbonate, -hydrogencarbonate, -hydroxycarbonate, - silikate, insbesondere -metasilikate, sowie Alkaliphosphate verwendet werden. In einer bevorzugten Ausführungsform enthalten die erfindungsgemäßen Blondiermittel mindestens zwei unterschiedliche Alkalisierungsmittel. Dabei können Mischungen beispielsweise aus einem Hydroxycarbonat und einem Metasilikat bevorzugt sein.

Die Zubereitungen enthalten Alkalisierungsmittel bevorzugt in Mengen von 1-25 Gew.-%, insbesondere 1,5-20 Gew.-%.

Ferner kann die erfindungsgemäße Zubereitung zusätzlich noch eine weitere Pflegekomponente als Haarstruktur-stabilisierenden Wirkstoff enthalten.

Beispiele für erfindungsgemäß bevorzugte, strukturverbessernde Wirkstoffe stellen Vitamine und deren Derivate beziehungsweise Vorstufen dar. Erfindungsgemäß besonders bevorzugt sind Panthenol und seine physiologisch verträglichen Derivate. Solche Derivate sind insbesondere die Ester und Ether des Panthenols sowie kationisch derivatisierte Panthenole. Einzelne Vertreter sind beispielsweise das Panthenoltriacetat, der Panthenolmonoethylether und dessen Monoacetat sowie die in der WO 92/13829 A1 offenbarten kationischen Panthenolderivate. Ein erfindungsgemäß bevorzugtes Panthenolderivat ist ferner dessen Vorstufe Pantolacton. Panthenol ist innerhalb dieser Gruppe bevorzugt.

Weiterhin ist auch Polyvinylpyrrolidon (PVP) für seine faserstrukturverbessernden Eigenschaften bekannt und erfindungsgemäß bevorzugt.

Weitere, erfindungsgemäß besonders wirksame, strukturverbessernde Verbindungen stellen die Aldehyde dar. Besonders bevorzugte Beispiel sind Formaldehyd und Formaldehyd-abspaltende Verbindungen, wie beispielsweise Methoxymethylester, Dimethylol (thio) harnstoffderivate, Oxazolidinderivate, N-Hydroxymethylmaleinimid, Hexamethylentetramin und seine Derivate, Hydantoinderivate, Pyridinium-substituierte Dimethylether, Imidazolidinylharnstoff-Derivate, Isothiazolinone, 2-Brom-2-nitropropandiol und 5-Brom-5-nitro-1,3-dioxan. Weitere besonders bevorzugte Aldehyde sind Acetaldehyd, Glyoxal, Glycerinaldehyd und Glutardialdehyd.

Eine weitere geeignete Gruppe von strukturverbessernden Wirkstoffen sind Pflanzenextrakte.

Üblicherweise werden diese Extrakte durch Extraktion der gesamten Pflanze hergestellt. Es kann aber in einzelnen Fällen auch bevorzugt sein, die Extrakte ausschließlich aus Blüten und/oder Blättern der Pflanze herzustellen.

Hinsichtlich der erfindungsgemäß verwendbaren Pflanzenextrakte wird insbesondere auf die Extrakte hingewiesen, die in der auf Seite 44 der 3. Auflage des Leitfadens zur Inhaltsstoffdeklaration kosmetischer Mittel, herausgegeben vom Industrieverband Körperpflege- und Waschmittel e.V. (IKW), Frankfurt, beginnenden Tabelle aufgeführt sind.

Erfindungsgemäß sind vor allem die Extrakte aus Eichenrinde, Brennessel, Hamamelis, Hopfen, Kamille, Klettenwurzel, Schachtelhalm, Weißdorn, Lindenblüten, Mandel, Aloe Vera, Fichtennadel, Roßkastanie, Sandelholz, Wacholder, Kokosnuß, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Meristem, grünem Tee, Ginseng und Ingwerwurzel bevorzugt.

Besonders bevorzugt sind die Extrakte aus Eichenrinde, Brennessel, Hamamelis, Hopfen, Kamille, Klettenwurzel, Schachtelhalm, Lindenblüten, Mandel, Aloe Vera, Kokosnuß, Mango, Aprikose, Limone, Weizen, Kiwi, Melone, Orange, Grapefruit, Salbei, Rosmarin, Birke, Wiesenschaumkraut, Quendel, Schafgarbe, Hauhechel, Meristem, grünem Tee, Ginseng und Ingwerwurzel.

Ganz besonders für die erfindungsgemäßen Mittel geeignet sind die Extrakte aus Mandel, Aloe Vera, Kokosnuß, Mango, Aprikose, Limone, Weizen, Kiwi, Melone und grünem Tee.

Als Extraktionsmittel zur Herstellung der genannten Pflanzenextrakte können Wasser, Alkohole sowie deren Mischungen verwendet werden. Unter den Alkoholen sind dabei niedere Alkohole wie Ethanol und Isopropanol, insbesondere aber mehrwertige Alkohole wie Ethylenglykol und Propylenglykol, sowohl als alleiniges Extraktionsmittel als auch in Mischung mit Wasser, bevorzugt. Pflanzenextrakte auf Basis von Wasser/Propylenglykol im Verhältnis 1:10 bis 10:1 haben sich als besonders geeignet erwiesen.

Die Pflanzenextrakte können erfindungsgemäß sowohl in reiner als auch in verdünnter Form eingesetzt werden. Sofern sie in verdünnter Form eingesetzt werden, enthalten sie üblicherweise ca. 2 - 80 Gew.-% Aktivsubstanz und als Lösungsmittel das bei ihrer Gewinnung eingesetzte Extraktionsmittel oder Extraktionsmittelgemisch.

Weiterhin kann es bevorzugt sein, in den erfindungsgemäßen Mitteln Mischungen aus mehreren, insbesondere aus zwei, verschiedenen Pflanzenextrakten einzusetzen.

Ebenfalls erfindungsgemäß als strukturverbessernde Wirkstoffe bevorzugt sind Honigextrakte. Diese Extrakte werden in analoger Weise zu den Pflanzenextrakten gewonnen und enthalten üblicherweise 1-10 Gew.-%, insbesondere 3-5 Gew.-%, Aktivsubstanz. Wasser/Propylenglykol-Mischungen können auch hier bevorzugte Extraktionsmittel sein.

Weitere strukturverbessernde Wirkstoffe sind Proteinhydrolysate, insbesondere Elastin-, Kollagen-, Keratin-, Milcheiweiß-, Sojaprotein-, Mandelprotein- und Weizenproteinhydrolysate, deren Kondensationsprodukte mit Fettsäuren sowie quaternisierte Proteinhydrolysate. Besonders bevorzugt sind stark abgebaute Keratinhydrolysate mit Molmassen im Bereich von 400 bis 800. Ferner sind quaternierte Proteinhydrolysate, wie sie beispielsweise unter den Handelsbezeichnungen Gluadin® WQ (INCI-Bezeichnung: Laurdimonium Hydroxypropyl Hydrolyzed Wheat Protein) und Crotein® Q (INCI-Bezeichnung: Hydroxypropyltrimonium Hydrolyzed Collagen) vertrieben werden, erfindungsgemäß besonders bevorzugt.

Neben den quaternierten Proteinhydrolysaten stellen auch quaternäre Polymere erfindungsgemäß bevorzugte strukturverbessernde Verbindungen dar. Besonders bevorzugt sind die Polymere, die unter den Handelsbezeichnungen Mirapol® A15 (INCI-Bezeichnung: Polyquaternium-2), Onamer® M (INCI-Bezeichnung: Polyquaternium-1) und Merquat® 100 (INCI-Bezeichnung: Polyquatemium-6) vertrieben werden.

Ebenfalls faserstrukturverbessernde Wirkstoffe sind Mono-, Di- und Oligosaccharide wie beispielsweise Glucose, Galactose, Fructose, Fruchtzucker, Saccharose und Lactose. Weiterhin können auch Derivate dieser Pentosen und Hexosen, wie die entsprechenden On- und Uronsäuren (Zuckersäuren), Zuckeralkohole, Zuckeramine, wie beispielsweise N-Glucosamin, und Glykoside, erfindungsgemäß eingesetzt werden. Die Zuckersäuren können erfindungsgemäß in freier Form, in Form ihrer Salze, bevorzugt sind Calcium-, Magnesium- und Zink-Salze, und in Form ihrer Ester oder Lactone eingesetzt werden. Bevorzugte Zuckersäuren sind die Gluconsäure, Gluconsäure-γ-lacton, Lactobionsäure, die Glucuronsäure und ihre Mono- beziehungsweise Dilactone, die Pangaminsäure, die Zuckersäure, die Mannozuckersäure und ihre Mono- beziehungsweise Dilactone sowie die Schleimsäure und ihre Mono- beziehungsweise Dilactone. Bevorzugte Zuckeralkohole sind Sorbit, Mannit und Dulcit. Bevorzugte Glykoside sind die Methylglucoside. Glucose, N-Glucosamin und Gluconsäure sind aus dieser Gruppe besonders bevorzugt.

Auch gewisse Aminosäuren sind im Rahmen der vorliegenden Erfindung als haarstrukturverbessernde Wirkstoffe einsetzbar. Beispiele sind die in der DE-195 22 569, auf die hier ausdrücklich Bezug genommen wird, beschriebenen Aminosäuren Serin, Threonin und Tyrosin. Ferner sind auch Derivate des Serins, wie beispielsweise das Serinphosphat, erfindungsgemäß bevorzugt. Weitere strukturverbessernde Aminosäuren stellen Lysin und Arginin dar. Serin und Arginin sind besonders bevorzugte faserstrukturverbessemde Wirkstoffe.

Ebenfalls zur Strukturverbesserung können bestimmte Säuren, insbesondere α-Hydroxycarbonsäuren, und ihre Salze eingesetzt werden. Erfindungsgemäß bevorzugte strukturverbessernde Säuren sind Milchsäure, Äpfelsäure, Weinsäure, Glycerinsäure und Maleinsäure. Milchsäure ist besonders bevorzugt. Weiterhin verbessern spezielle Phosphonsäuren und ihre Salze die Struktur keratinischer Fasern. Erfindungsgemäß bevorzugte Phosphonsäuren sind die n-Octylphosphonsäure und die n-Decylphosphonsäure.

Weiterhin sind lipidlösliche Esteralkohole oder Esterpolyole für ihre strukturverbessernde Wirkung bekannt. Als lipidlöslich sind sie dann anzusehen, wenn sich 5 Gew.-% dieser Produkte in Cetylalkohol bei 80° C klar auflösen.

Die erfindungsgemäß geeigneten Esteralkohole oder Esterpolyole sind erhältlich durch Umsetzung eines Epoxyfettsäureesters mit Wasser oder ein- oder mehrwertigen Alkoholen mit 1-10 C-Atomen unter Öffnung des Epoxidrings und Ausbildung einer vicinalen Dihydroxyethyl- oder Hydroxy-alkoxy-ethylgruppe. Der Epoxyfettsäureester kann dabei auch ein Epoxidationsprodukt aus einem technischen Fettsäureester mit Anteilen gesättigter Fettsäuren sein. Der Epoxidsauerstoffgehalt sollte aber wenigstens 3 Gew.-%, bevorzugt 5-10 Gew.-%, betragen.

Die Epoxyfettsäureester sind dabei entweder epoxidierte Fettsäureester einwertiger Alkohole, also z.B. epoxidierter Ölsäuremethylester, Linolsäuremethylester, Ricinolsäuremethylester oder epoxidierte Fettsäureester mehrwertiger Alkohole, z.B. Glycerinmonooleat oder Propylenglycol-monooleat oder epoxidierte Fettsäuretriglyceride, z.B. Ölsäuretriglycerid oder ungesättigte Öle wie z.B. Olivenöl, Sojaöl, Sonnenblumenöl, Leinöl, Rüböl.

Technisch besonders interessant sind vor allem ungesättigte Fettsäuremethylester-Epoxide aus ungesättigten Pflanzenfettsäuren. So ist als Esterpolyol das Umsetzungsprodukt eines Pflanzenölfettsäuremethylester-Epoxidats mit einem Polyol mit 2-6 C-Atomen und 2 - 6 Hydroxylgruppen besonders bevorzugt. Als Polyole können dabei z.B. Ethylenglycol, 1,2-Propylenglycol, 1,3-Propylenglycol, Butandiol, Pentandiol, Hexandiol, Glycerin, Trimethylolpropan, Pentaerythrit, Sorbit oder Diglycerin enthalten sein.

Besonders gut eignet sich dabei für die erfindungsgemäßen Haarbehandlungsmittel als Esterpolyol das Umsetzungsprodukt eines Pflanzenfettsäuremethylester-Epoxidats mit Trimethylpropan und mit einer Hydroxylzahl von 350 - 450. Ein solches Produkt auf Basis von Sojaölfettsäuremethylesier-Epoxid und Trimethylolpropan ist unter der Handelsbezeichnung Sovermol®760 erhältlich.

Weiterhin kann als strukturverbessernder Wirkstoff Vitamin B₃ eingesetzt werden. Unter dieser Bezeichnung werden häufig die Verbindungen Nicotinsäure und Nicotinsäureamid (Niacinamid) geführt. Erfindungsgemäß bevorzugt ist das Nicotinsäureamid.

Auch Vitamin H ist als strukturverbessernder Wirkstoff im Sinne der vorliegenden Erfindung einsetzbar. Als Vitamin H wird die Verbindung (3a*S*,4*S*, 6a*R*)-2-Oxohexahydrothienol[3,4-*d*]-imidazol-4-valeriansäure bezeichnet, für die sich aber zwischenzeitlich der Trivialname Biotin durchgesetzt hat.

Erfindungsgemäß besonders bevorzugte strukturverbessernde Wirkstoffe sind ausgewählt aus Panthenol, physiologisch verträglichen Panthenol-Derivaten, Mono-, Di- und Oligosacchariden, Serin, Arginin, Niacinamid, Polyvinylpyrrolidon, Gluconsäure, Biotin und den lipidlöslichen Esteralkoholen oder Esterpolyolen.

Die erfindungsgemäßen Mitttel enthalten die zusätzlichen strukturverbessernden Wirkstoffe bevorzugt in Mengen von 0,1 bis 5 Gew.-%, besonders bevorzugt in Mengen von 0,2 bis 2 Gew.-%.

Die erfindungsgemäßen Zubereitungen können als Feststoff vorliegen oder können in einen geeigneten wäßrigen, alkoholischen oder wäßrig-alkoholischen Träger eingearbeitet sein. Zum Zwecke der Blondierung sind solche Träger beispielsweise Cremes, Emulsionen, Gele, Pasten, oder andere Zubereitungen, die für die Anwendung auf dem Haar geeignet sind. Unter wäßrig-alkoholischen Lösungen sind im Sinne der vorliegenden Erfindung wäßrige Lösungen enthaltend 3 bis 70 Gew.-% eines C₁-C₄-Alkohols, insbesondere Ethanol bzw. Isopropanol, zu verstehen. Die erfindungsgemäßen Mittel können zusätzlich weitere organische Lösemittel, wie beispielsweise Methoxybutanol, Benzylallcohol, Ethyldiglykol oder 1,2-Propylenglykol, enthalten. Bevorzugt sind dabei alle wasserlöslichen organischen Lösemittel.

Zum Blondieren menschlicher Haare - insbesondere für die Strähnchenapplikation - werden üblicherweise feste oder pastenförmige Zubereitungen mit festen Oxidationsmitteln unmittelbar vor der Anwendung mit einer verdünnten Wasserstoffperoxid-lösung vermischt. Diese Mischung wird dann auf das Haar aufgebracht und nach einer bestimmten Einwirkzeit wieder ausgespült. Die Konzentration dieser Wasserstoffperoxid-Lösung wird einerseits von den gesetzlichen Vorgaben und andererseits von dem gewünschten Effekt bestimmt; in der Regel werden 6- bis 12-prozentige Lösungen in Wasser verwendet. Die Mengenverhältnisse von Blondiermittel und Wasserstoffperoxid-Lösung liegen dabei üblicherweise im Bereich von 1:1 bis 1:2, wobei ein Überschuß an Wasserstoffperoxid-Lösung insbesondere dann gewählt wird, wenn eine ausgeprägte Blondierwirkung erwünscht ist.

Wird die Zubereitung als Pulver konfektioniert, enthalten sie in der Regel zusätzlich eine Komponente zur Entstaubung der feinpulverisierten Formulierung. Solche Entstaubungsmittel sind üblicherweise Öle, flüssige Wachse, Etherderivate. In der Druckschrift WO 00/30596 werden bei 25°C flüssige Lösemittel, ausgewählt aus der Gruppe der Kohlenwasserstoffe, der Alkohole, der Ester sowie der Ketone, wie z.B. 3-Methoxybutanol, Benzylalkohol, 1,2-Propandiol, Hexanol, Cyclohexanon, Propylencarbonat und Ethyldiglykol offenbart, die sich zur Entstaubung eignen und in den erfindungsgemäßen Zubereitungen enthalten sein können.

Die erfindungsgemäßen Mittel können weiterhin alle für solche Zubereitungen bekannten Wirk-, Zusatz- und Hilfsstoffe enthalten. In vielen Fällen enthalten die Zubereitungen mindestens ein Tensid, wobei prinzipiell sowohl anionische als auch zwitterionische, ampholytische, nichtionische und kationische Tenside geeignet sind. In vielen Fällen hat es sich aber als vorteilhaft erwiesen, die Tenside aus anionischen, zwitterionischen oder nichtionischen Tensiden auszuwählen.

Als anionische Tenside eignen sich in den erfindungsgemäßen Zubereitungen alle für die Verwendung am menschlichen Körper geeigneten anionischen oberflächenaktiven Stoffe. Diese sind gekennzeichnet durch eine wasserlöslichmachende, anionische Gruppe wie z. B. eine Carboxylat-, Sulfat-, Sulfonat- oder Phosphat-Gruppe und eine lipophile Alkylgruppe mit etwa 10 bis 22 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 oder 3 C-Atomen in der Alkanolgruppe,
- lineare Fettsäuren mit 10 bis 22 C-Atomen (Seifen),
- Ethercarbonsäuren der Formel R-O-(CH₂-CH₂O)ₓ -CH₂-COOH, in der R eine lineare Alkylgruppe mit 10 bis 22 C-Atomen und x = 0 oder 1 bis 16 ist,
- Acylsarcoside mit 10 bis 18 C-Atomen in der Acylgruppe,
- Acyltauride mit 10 bis 18 C-Atomen in der Acylgruppe,
- Acylisethionate mit 10 bis 18 C-Atomen in der Acylgruppe,
- Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen,
- lineare Alkansulfonate mit 12 bis 18 C-Atomen,
- lineare Alpha-Olefinsulfonate mit 12 bis 18 C-Atomen,
- Alpha-Sulfofettsäuremethylester von Fettsäuren mit 12 bis 18 C-Atomen,
- Alkylsulfate und Alkylpolyglykolethersulfate der Formel R-O(CH₂-CH₂O)ₓ-SO₃H, in der R eine bevorzugt lineare Alkylgruppe mit 10 bis 18 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 12 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül sowie insbesondere Salze von gesättigten und insbesondere ungesättigten C₈-C₂₂-Carbonsäuren, wie Ölsäure, Stearinsäure, Isostearinsäure und Palmitinsäure.

Als zwitterionische Tenside werden solche oberflächenaktiven Verbindungen bezeichnet, die im Molekül mindestens eine quartäre Ammoniumgruppe und mindestens eine -COO⁽⁻ ⁾_ oder -SO₃⁽⁻⁾-Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammonium-glycinate, beispielsweise das Kokosacylaminopropyl-dimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Unter ampholytischen Tensiden werden solche oberflächenaktiven Verbindungen verstanden, die außer einer C₈₋₁₈-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂₋₁₈-Acylsarcosin.

Nichtionische Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂₋₂₂-Fettsäuremono- und -diester von Anlagerungsprodukten von 1 bis 30 Mol Ethylenoxid an Glycerin,
- C₈₋₂₂-Alkylmono- und -oligoglycoside und deren ethoxylierte Analoga,
- Anlagerungsprodukte von 5 bis 60 Mol Ethylenoxid an Rizinusöl und gehärtetes Rizinusöl,
- Anlagerungeprodukte von Ethylenoxid an Sorbitanfettsäureester
- Anlagerungsprodukte von Ethylenoxid an Fettsäurealkanolamide.

Beispiele für die in den erfindungsgemäßen Formulierungen verwendbaren kationischen Tenside sind insbesondere quartäre Ammoniumverbindungen. Bevorzugt sind Ammoniumhalogenide wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Weitere erfindungsgemäß verwendbare kationische Tenside stellen die quaternisierten Proteinhydrolysate dar.

Erfindungsgemäß ebenfalls geeignet sind kationische Silikonöle wie beispielsweise die im Handel erhältlichen Produkte Q2-7224 (Hersteller: Dow Coming; ein stabilisiertes Trimethylsilylamodimethicon), Dow Corning 929 Emulsion (enthaltend ein hydroxylamino-modifiziertes Silicon, das auch als Amodimethicone bezeichnet wird), SM-2059 (Hersteller: General Electric), SLM-55067 (Hersteller: Wacker) sowie Abil®-Quat 3270 und 3272 (Hersteller: Th. Goldschmidt; diquaternäre Polydimethylsiloxane, Quaternium-80).

Alkylamidoamine, insbesondere Fettsäureamidoamine wie das unter der Bezeichnung Tego Amid®S 18 erhältliche Stearylamidopropyldimethylamin, zeichnen sich neben einer guten konditionierenden Wirkung speziell durch ihre gute biologische Abbaubarkeit aus. Ebenfalls sehr gut biologisch abbaubar sind quaternäre Esterverbindungen, sogenannte "Esterquats", wie die unter dem Warenzeichen Stepantex® vertriebenen Methylhydroxyalkyldialkoyloxyalkyl-ammoniummethosulfate sowie die unter dem Warenzeichen Dehyquart® vertriebenen Produkte wie Dehyquart® AU-46.

Ein Beispiel für ein als kationisches Tensid einsetzbares quaternäres Zuckerderivat stellt das Handelsprodukt Glucquat®100 dar, gemäß INCI-Nomenklatur ein "Lauryl Methyl Gluceth-10 Hydroxypropyl Dimonium Chloride".

Bei den als Tenside eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Weiterhin können die erfindungsgemäßen Zubereitungen bevorzugt noch einen konditionierenden Wirkstoff, ausgewählt aus der Gruppe, die von kationischen Tensiden, kationischen Polymeren, Alkylamidoaminen, Paraffinölen, pflanzlichen Ölen und synthetischen Ölen gebildet wird, enthalten.

Als konditionierende Wirkstoffe bevorzugt sein können kationische Polymere. Dies sind in der Regel Polymere, die ein quartäres Stickstoffatom, beispielsweise in Form einer Ammoniumgruppe, enthalten.
Bevorzugte kationische Polymere sind beispielsweise
- quaternisierte Cellulose-Derivate, wie sie unter den Bezeichnungen Celquat® und Polymer JR® im Handel erhältlich sind. Die Verbindungen Celquat® H 100, Celquat® L 200 und Polymer JR®400 sind bevorzugte quaternierte Cellulose-Derivate.
- polymere Dimethyldiallylammoniumsalze und deren Copolymere mit Acrylsäure sowie Estern und Amiden von Acrylsäure und Methacrylsäure. Die unter den Bezeichnungen Merquat®100 (Poly(dimethyldiallylammoniumchlorid)), Merquat®550 (Dimethyldiallylammoniumchlorid-Acrylamid-Copolymer) und Merquat® 280 (Dimethyldiallylammoniumchlorid-Acrylsäure-Copolymer im Handel erhältlichen Produkte sind Beispiele für solche kationischen Polymere.
- Copolymere des Vinylpyrrolidons mit quaternierten Derivaten des Dialkylaminoacrylats und -methacrylats, wie beispielsweise mit Diethylsulfat quaternierte Vinylpyrrolidon-Dimethylaminomethacrylat-Copolymere. Solche Verbindungen sind unter den Bezeichnungen Gafquat®734 und Gafquat®755 im Handel erhältlich.
- Vinylpyrrolidon-Methoimidazoliniumchlorid-Copolymere, wie sie unter der Bezeichnung Luviquat® angeboten werden.
- quaternierter Polyvinylalkohol
   sowie die unter den Bezeichnungen
- Polyquaternium 2,
- Polyquaternium 17,
- Polyquaternium 18 und
- Polyquaternium 27 bekannten Polymeren mit quartären Stickstoffatomen in der Polymerhauptkette.

Besonders bevorzugt sind kationische Polymere der vier erstgenannten Gruppen, ganz besonders bevorzugt sind Polyquaternium-2, Polyquaternium-10 und Polyquaternium-22.

Alternativ zu den kationischen Polymeren werden als konditionierende Wirkstoffe zwitterionische oder ampholytische Polymere besonders bevorzugt eingesetzt. Bevorzugte Vertreter sind Octylacrylamid/Methylmethacrylat/tert-Butylaminoethylmethacrylat/2-Hydroxypropylmethacrylat Copolymere und insbesondere das Acrylamidopropyl-trimethylammoniumchlorid/Acrylat Copolymer.

Als konditionierende Wirkstoffe weiterhin geeignet sind Silikonöle, insbesondere Dialkyl- und Alkylarylsiloxane, wie beispielsweise Dimethylpolysiloxan und Methylphenylpolysiloxan, sowie deren alkoxylierte und quaternierte Analoga. Beispiele für solche Silikone sind die von Dow Corning unter den Bezeichnungen DC 190, DC 200, DC 344, DC 345 und DC 1401 vertriebenen Produkte sowie die Handelsprodukte Q2-7224 (Hersteller: Dow Corning; ein stabilisiertes Trimethylsilylamodimethicon), Dow Corning® 929 Emulsion (enthaltend ein hydroxyl-amino-modifiziertes Silicon, das auch als Amodimethicone bezeichnet wird), SM-2059 (Hersteller: General Electric), SLM-55067 (Hersteller: Wacker) sowie Abil®-Quat 3270 und 3272 (Hersteller: Th. Goldschmidt; diquaternäre Polydimethylsiloxane, Quaternium-80).

Ebenfalls einsetzbar als konditionierende Wirkstoffe sind Paraffinöle, synthetisch hergestellte oligomere Alkene sowie pflanzliche Öle wie Jojobaöl, Sonnenblumenöl, Orangenöl, Mandelöl, Weizenkeimöl und Pfirsichkemöl.

Gleichfalls geeignete haarkonditionierende Verbindungen sind Phospholipide, beispielsweise Sojalecithin, Ei-Lecithin und Kephaline.

Weitere Wirk-, Hilfs- und Zusatzstoffe sind beispielsweise
- nichtionische Polymere wie beispielsweise Vinylpyrrolidon/Vinylacrylat-Copolymere, Polyvinylpyrrolidon und Vinylpyrrolidon/Vinylacetat-Copolymere und Polysiloxane,
- anionische Polymere wie beispielsweise Polyacrylsäuren, vemetzte Polyacrylsäuren, Vinylacetat/Crotonsäure-Copolymere, Vinylpyrrolidon/Vinylacrylat-Copolymere, Vinylacetat/Butylmaleat/Isobornylacrylat-Copolymere, Methylvinylether/Maleinsäureanhydrid-Copolymere und Acrylsäure/Ethylacrylat/N-tert.Butyl-acrylamid-Terpolymere,
- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z.B. Polyvinylalkohol,
- Strukturanten wie Maleinsäure und Milchsäure,
- Parfümöle, Dimethylisosorbid und Cyclodextrine,
- Lösungsmittel und -vermittler wie Ethanol, Isopropanol, Ethylenglykol, Propylenglykol, Glycerin und Diethylenglykol,
- quaternierte Amine wie Methyl-1-alkylamidoethyl-2-alkylimidazolinium-methosulfat
- Entschäumer wie Silikone,
- Farbstoffe zum Anfärben der Zubereitung,
- Antischuppenwirkstoffe wie Piroctone Olamine, Zink Omadine und Climbazol,
- Lichtschutzmittel, insbesondere derivatisierte Benzophenone, Zimtsäure-Derivate und Triazine,
- Substanzen zur Einstellung des pH-Wertes, wie beispielsweise übliche Säuren, insbesondere Genußsäuren und Basen,
- Wirkstoffe wie Allantoin, Pyrrolidoncarbonsäuren und deren Salze sowie Bisabolol,
- Vitamine, Provitamine und Vitaminvorstufen, insbesondere solche der Gruppen A, B₃, B₅, C, E, F und H,
- Cholesterin,
- Konsistenzgeber wie Zuckerester, Polyolester oder Polyolalkylether,
- Fette und Wachse wie Walrat, Bienenwachs, Montanwachs und Paraffine,
- Fettsäurealkanolamide,
- Komplexbildner wie EDTA, NTA, β-Alanindiessigsäure und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex, Styrol/PVP- und Styrol/Acrylamid-Copolymere
- Perlglanzmittel wie Ethylenglykolmono- und -distearat sowie PEG-3-distearat,
- Pigmente,
- Stabilisierungsmittel für Wassserstoffperoxid und andere Oxidationsmittel,
- Treibmittel wie Propan-Butan-Gemische, N₂O, Dimethylether, CO₂ und Luft,

Bezüglich weiterer fakultativer Komponenten sowie die eingesetzten Mengen dieser Komponenten wird ausdrücklich auf die dem Fachmann bekannten einschlägigen Handbücher, z. B. Kh. Schrader, Grundlagen und Rezepturen der Kosmetika, 2. Auflage, Hüthig Buch Verlag, Heidelberg, 1989, verwiesen.

Ein dritter Gegenstand der vorliegenden Erfindung ist ein Verfahren zur Blondierung keratinischer Faserrn, in dem
- gewünschtenfalls ein Vorbehandlungsmittel M1 auf die Faser aufgebracht wird, dann
- ein Blondiermittel M2, enthaltend mindestens ein Oxidationsmittel, auf der Faser zur Anwendung kommt, wobei gewünschtenfalls dem Mittel M2 unmittelbar vor der Anwendung ein weiteres Mittel M3 zugegeben wird,
- dieses Blondiermittel M2 nach einer Zeit von 5-30 Minuten von der Faser abgespült wird
- und nach der Behandlung gegebenenfalls ein Mittel M4 auf die Faser aufgetragen und nach einer Einwirkzeit von einigen Minuten wieder abgespült wird,
wobei mindestens eines der Mittel M1, M2, M3 oder M4 mindestens ein Vitamin B6-Derivat, ausgewählt aus der gruppe Pyridoxin, Pyridoxal, Pyridoxamin, oder Pyridoxal-5'-phosphat enthält.

Das Mittel M1 ist ein Vorbehandlungsmittel, enthaltend mindestens eine Verbindung gemäß Formel (I) in einem kosmetischen Träger. Das Blondiermittel M2 kann neben mindestens einem Oxidationsmittel die oben genannten Inhaltsstoffe enthalten. Es ist bevorzugt, dem Mittel M2 kurz vor der Anwendung ein Mittel M3, enthaltend mindestens eine Verbindung gemäß Formel (I), zuzufügen. Die daraus resultierende Mischung entspricht der erfindungsgemäßen Zubereitung. Das Mittel M3, enthaltend mindestens eine Verbindung der Formel (I), kann sowohl als Feststoff als auch als wäßrige Lösung konfektioniert sein.
Das Mittel M4 ist erfindungsgemäß ein Haarnachbehandlungsmittel, wie es z.B. in K. Schrader, Grundlagen und Rezepturen der Kosmetika, 2. Auflage, 1989, Dr. Alfred Hüthig Verlag, Heidelberg S. 722 ff. beschrieben wird, welches gegebenenfalls mindestens ein Vitamin B6-Derivat, ausgewählt aus der gruppe Pyridoxin, Pyridoxal, Pyridoxamin, oder Pyridoxal-5'-phosphat enthält.

Die folgenden Beispiele sollen den Gegenstand der vorliegenden Anmeldung verdeutlichen.

### Beispiele

### 1) Herstellung der Blondiercremes

Es wurde eine Referenz-Blondiercreme (Creme 1) ohne Vitamin B6 (Pyridoxin·HCl) und eine Blondiercreme (Creme 2) mit Vitamin B6 hergestellt (siehe Tabelle 1).

**Tabelle 1:**

| (alle Angaben in Gew.%) | | |
|---|---|---|
| | Creme 1 | Creme 2 (erfindungsgemäß) |
| Hydrenol®D¹ | 15% | 15 % |
| Kokoslorol®C12-18² | 2 % | 2 % |
| Eumulgin®B2³ | 0.75 % | 0.75 % |
| Texapon®NSO⁴ | 5 % | 5 % |
| Dehyton®K⁵ | 3.75 % | 3.75 % |
| Pyridoxin·HCl | - | 2.0 % |
| L-Arginin | 1.0 % | 1.0 % |
| Gluadin® W40⁶ | 1.0% | 1.0 % |
| Natriumsilikat | 0.2 % | 0.2 % |
| Ammoniak⁷ | ad pH 10 | ad pH 10 |
| Wasser | ad 100 % | ad 100 % |

| | | |
|---|---|---|
| ¹ C₁₆-C₁₈-Fettalkohol (INCI-Bezeichnung: Cetearyl alcohol) (COGNIS) | | |
| ² C₁₂-C₁₈-Fettalkohol (INCI-Bezeichnung: Coconut alcohol) (COGNIS) | | |
| ³ Cetearylstearylalkohol mit ca. 20 EO-Einheiten (INCI-Bezeichnung: Ceteareth-20) (COGNIS) | | |
| ⁴ Laurylethersulfat, Natriumsalz mit ca. 2 EO-Einheiten (ca. 27,5 % Aktivsubstanz; (INCI-Bezeichnung: Sodium Laureth Sulfate) (COGNIS) | | |
| ⁵ N,N-Dimethyl-N-(C₈-C₁₈-kokosamidopropyl)ammoniumacetobetain (ca. 30 % Aktiv-substanz; INCI-Bezeichnung: Aqua (Water), Cocamidopropyl Betaine) (COGNIS) | | |
| ⁶ Weizenproteinhydrolysat, (INCI-Bezeichnung: Aqua (Water), Hydrolyzed Wheat Protein, Sodium Benzoate) Phenoxyethanol, Methylparaben, Propylparaben) (COGNIS) | | |
| ⁷ 25 %-ige wäßrige Lösung | | |

Die Substanzen Hydrenol®D, Kokoslorol®C12-18, Eumulgin®B2, Texapon®NSO, Gluadin® W40 und Dehyton®K wurden bei 80°C aufgeschmolzen, mit dem 80°C heißem Wasser vermischt und unter starkem Rühren emulgiert. Danach wurde die Emulsion unter schwachem Rühren abgekühlt. Bei einer Temperatur von 40°C wurden die restlichen Komponenten unter Rühren zu der Emulsion gegeben.

### 2) Nachweis der strukturgebenden Wirkung von Vitamin B6 bei gleichzeitiger Applikation von Vitamin B6 mit dem Blondiermittel

### A) Verwendete Analysemethode: HP-DSC (High Pressure Differential Scanning Calorimetry)

Thermoanalytische Untersuchungen eignen sich besonders zur Charakterisierung von Zweiphasensystemen, zu denen die Humanhaare als Faserkeratine mit ihrem kristallinen α-Helix-Anteil und amorphen Matrix-Anteil ebenfalls gehören. Auf der einen Seite können Glasübergänge und Alterungsverhalten der amorphen Matrix untersucht werden, auf der anderen Seite liefert das Schmelzverhalten der kristallinen, helicalen Phase wichtige Erkenntnisse. Thermoanalytische Untersuchungen sind erstmals 1899 beschrieben, erste Differenzthermoanalysen (DTA) an Proteinfasem wurden Ende der fünfziger Jahre durchgeführt (F. Schwenker, J.H. Dusenbury, Text. Res. J. 1963, 30, Seite 800 ff; W. D. Felix, M.A. McDowall, H. Eyring, ibid. (1963), 33, Seite 465 ff.). In den folgenden Jahren sind unterschiedliche thermoanalytische Meßverfahren, wie DTA, HP-DTA (High Pressure, Hochdruck-DTA) und DSC (Differential Scanning Calorimetry, Dynamische Differenz-Kalorimetrie), an Keratinfasern angewendet worden um z.B. das Phänomen der Superkontraktion, α-β-Phasenübergänge der Helices oder Denaturierungsvorgänge zu untersuchen. In jüngster Zeit wird, insbesondere am Deutschen Wollforschungsinstitut in Aachen (F.J. Wortmann, H. Deutz, J. Appl. Polym.

Sci. 1993, 48, Seite 137ff.), die Methode der IIP-DSC zur Untersuchung von Keratinfasern genutzt, die die Probleme mit pyrolytischen Effekten, wie sie bei der konventionellen DSC auftreten, und Probleme mit der Datenerfassung und -interpretation, wie sie die DTA birgt, ausschließt. Dabei werden DSC-Messungen an Keratinen durchgeführt, die mit Wasser in kommerziell erhältlichen, druckfesten Meßkapseln eingeschlossen sind. Im Keratin-Wasser-System entwickelt sich beim Erhitzen oberhalb von 100°C in den verkapselten Stahltiegeln ein Wasserdampfhochdruck, woraus sich die HP-DSC Analyse ableitet. Der gravierende Unterschied der HP-DSC-Thermogramme von Humanhaaren im Vergleich zu normalen DSC-Thermogrammen ist der, daß die endothermen Peaks, die den Umwandlungspunkt und Umwandlungsenthalpie wiedergeben, hier um ca. 90 C zu niedrigeren Temperaturen verschoben sind. Das rührt daher, daß das Wasser nach Diffusion in die Haarfaser durch Schwächung und Spaltung von Wasserstoffbrücken- und Salzbindungen die Proteinstabilität vermindert und so die "Verleimungstemperatur" der Keratine herabsetzt. Werden durch das superkontrahierende Agens wie Wasser nur Wasserstoffbrücken und Salzbrücken gelöst, so ist der thermische Effekt reversibel (Superkontraktion). Der Vorgang wird jedoch irreversibel, sobald auch kovalente Bindungen, wie z. B. Disulfidbrücken, gespalten werden. Dies tritt ein, wenn man Humanhaarfasern in druckfesten Kapseln mit Wasser auf über 150 °C erhitzt. Die irreversible Umwandlung, interpretiert als Übergang der α-helicalen Bereiche in den Proteinen in einen ungeordneten Zustand, resultiert in endothermen Peaks, wobei die Peaklage den Umwandlungs- oder auch Denaturierungspunkt und die Peakfläche die Umwandlungs- oder Denaturierungs-Enthalpie wiedergibt.

Unter Verwendung der Dynamischen Differenz-Kalorimetrie (DSC) können demnach strukturelle sowie chemische Zustände und Veränderungen in Faserkeratinen und insbesondere in Humanhaaren erfaßt werden. Unter genau definierten Versuchsbedingungen kann man bei Humanhaaren die kalorimetrisch erfaßbaren Vorgänge anhand von Thermogrammen aufzeichnen und sie bezüglich der Peaklagen, - strukturen und -flächen als Indikator für die Beeinflussung von Ordnungs-Unordnungsübergangen durch Änderungen innerer und/oder äußerer Parameter, hervorrufen z. B. durch kosmetische Behandlung der Haare, verwenden. D. h. aus den im Thermogramm von Humanhaaren aufgezeichneten endothermen Peaks lassen sich aufgrund von Peaklage (Umwandlungspunkt) und Peakfläche (Umwandlungsenthalpie) Aussagen über Festigkeit bzw. Schädigung der Humanhaarfaser treffen.

Ausführliche Untersuchungen bezüglich des Einflusses des Cystingehaltes auf die Denaturierung der α-Helices in Keratinen haben z. B. gezeigt, daß die Denaturierungs-Temperatur (Übergangstemperatur) des Keratins linear mit dem Cystingehalt ansteigt. Die erhöhte Stabilität des Matrixbereichs aufgrund des höheren Vemetzungsgrades des erhöhten Anteils an Disulfidbrücken in der Matrix führt dazu, daß die Umwandlung der in diese Matrix eingebetteten Helices erschwert wird und resultiert somit in einer Erhöhung der Denaturierungs-Temperatur. Umgekehrt kann in der Regel eine Denaturierungs-Temperatur- und vor allem Denaturierungs-Enthalpieerniedrigung bei durch Dauerwelle oder Bleichung bzw. Färbung behandelten Humanhaaren beobachtet werden (H. Deutz, Doktorarbeit, RWTH Aachen 1993).

### B) Durchführung

50 mL der jeweiligen Cremes 1 bzw. 2 wurden vor Ihrer Applikation auf eine Humanhaar-Strähne (Klugmann, 6622D, mittelblond) mit je 40 mL einer wäßrigen H₂O₂-Lösung vermischt. Im Experiment 2.3 und 2.4 wurden der Blondierformulierung vor der Applikation zusätzlich 10 g eines handelsüblichen Boosters (96 Gew-% Ammoniumperoxodisulfat und 4 Gew% Siliciumdioxid (amorph)) zugesetzt. Alle Formulierungen haben einen Misch-pH-Wert von 9.8-10.2. Nach einer Einwirkungszeit von 30 Minuten wurde das Haar gespült und getrocknet.
Die Denaturierungstemperaturen der blondierten Haarproben sowie des unbehandelten Haars wurden per HP-DSC thermoanalytisch bestimmt. Zusätzlich wurde mittels colorimetrischer Analyse die Aufhell-Leistung ermittelt.
Die Ergebnisse der HP-DSC Messung und der farbmetrischen Bestimmung des Aufhelleffekts der jeweiligen Formulierungen sind der Tabelle 2 zu entnehmen. Die aufgeführte CIELAB-Koordinate L ist ein Maß für die Helligkeit und berechnet sich aus den Normfarbwerten X, Y, Z, welche sich wiederum aus den Spektralverteilungen des Reflexionsgrades der Probe ergeben (H. G. Völz, Industrielle Farbprüfung, VCH, Weinheim, 1990.). Je größer der Wert für L ist, umso besser ist die Aufhelleistung des Blondiermittels.

**Tabelle 2:**

| | | **Denaturierungstemperatur** | |
|---|---|---|---|
| **Beispiel** | **Mischung** | **[°C]** | **L** |
| 2.0 | keine (unbehandeltes Haar) | 144.6 | 36.15 |
| 2.1 | Creme 1 / H₂O₂ (6 %ig) | 141.9 | 43.77 |
| 2.2 | Creme 2 / H₂O₂ (6 %ig) | 144.6 | 44.80 |
| 2.3 | Creme 1 / H₂O₂ (12 %ig)/ | 139.5 | 53.45 |
| | 10 g Booster | | |
| 2.4 | Creme 2 / H₂O₂ (12 %ig)/ | 142.9 | 54.12 |
| | 10 g Booster | | |

## Patentansprüche

1. Verwendung von Vitamin B6-Derivaten, ausgewählt aus der Gruppe Pyridoxin, Pyridoxal, Pyridoxamin oder Pyridoxal-5'-phosphat oder eines der entsprechenden physiologisch verträglichen Salze, zur Verringerung der Schädigung keratinischer Fasern, insbesondere menschlicher Haare, während der oxidativen Haarbehandlung.

2. Zubereitungen zur oxidativen Behandlung von Keratinfasem, insbesondere zur Haarblondierung, enthaltend mindestens ein Oxidationsmittel, **dadurch gekennzeichnet, daß** sie mindestens ein Vitamin B6-Derivat, ausgewählt aus der Gruppe Pyridoxin, Pyridoxal, Pyridoxamin oder Pyridoxal-5'-phosphat oder eines der entsprechenden physiologisch verträglichen Salze, enthalten.

3. Zubereitungen nach Anspruch 2, **dadurch gekennzeichnet, daß** sie die Vitamin B6-Derivate in einer Menge von 0.05-2 Gew.% bezogen auf die gesamte Zubereitung enthalten.

4. Zubereitungen nach einem der Ansprüche 2 oder 3, **dadurch gekennzeichnet, daß** sie zusätzlich eine Boosterkomponente enthalten.

5. Verfahren zur Blondierung keratinischer Fasern, in dem
gewünschtenfalls ein Vorbehandlungsmittel M1 auf die Faser aufgebracht wird, dann
- ein Blondiermittel M2, enthaltend mindestens ein Oxidationsmittel, auf der Faser zur Anwendung kommt, wobei gewünschtenfalls dem Mittel M2 unmittelbar vor der Anwendung ein weiteres Mittel M3 zugegeben wird,
- dieses Blondiermittel M2 nach einer Zeit von 5-30 Minuten von der Faser abgespült wird
- und nach der Behandlung gegebenenfalls ein Mittel M4 auf die Faser aufgetragen und nach einer Einwirkzeit von einigen Minuten wieder abgespült wird,
**dadurch gekennzeichnet, daß** mindestens eines der Mittel M1, M2, M3 oder M4 mindestens ein Vitamin B6-Derivat, ausgewählt aus der Gruppe Pyridoxin, Pyridoxal, Pyridoxamin oder Pyridoxal-5'-phosphat oder eines der entsprechenden physiologisch verträglichen Salze, enthält.

6. Verfahren nach Anspruch 5, **dadurch gekennzeichnet, daß** dem Mittel M2 kurz vor der Anwendung ein Mittel M3, enthaltend mindestens ein Vitamin B6-Derivat, ausgewählt aus der Gruppe Pyridoxin, Pyridoxal, Pyridoxamin oder Pyridoxal-5'-phosphat oder eines der entsprechenden physiologisch verträglichen Salze, zugegeben wird.

7. Verfahren nach einem der Ansprüche 5 oder 6, **dadurch gekennzeichnet, daß** das Mittel M2 eine Zubereitung gemäß einem der Ansprüche 2 bis 4 ist.

## Claims

1. Use of vitamin B6 derivatives chosen from the group consisting of pyridoxine, pyridoxal, pyridoxamine or pyridoxal-5'-phosphate or one of the corresponding physiologically compatible salts for reducing the damage to keratin fibres, in particular human hair, during the oxidative treatment of hair.

2. Preparations for the oxidative treatment of keratin fibres, in particular for bleaching hair, comprising at least one oxidizing agent, **characterized in that** they comprise at least one vitamin B6 derivative chosen from the group consisting of pyridoxine, pyridoxal, pyridoxamine or pyridoxal-5'-phosphate or one of the corresponding physiologically compatible salts.

3. Preparations according to Claim 2, **characterized in that** they comprise the vitamin B6 derivatives in an amount of 0.05-2% by weight, based on the total preparation.

4. Preparations according to one of Claims 2 or 3, **characterized in that** they additionally comprise a booster component.

5. Method of bleaching keratin fibres in which
if desired a pre-treatment agent M1 is applied to the fibres, then
- a bleaching agent M2 comprising at least one oxidizing agent is used on the fibres, where, if desired, a further agent M3 is added to the agent M2 directly prior to use,
- this bleaching agent M2 is rinsed off from the fibres after a time of 5-30 minutes
- and after the treatment where appropriate an agent M4 is applied to the fibres and rinsed off again after a contact time of a few minutes,
**characterized in that** at least one of the agents M1, M2, M3 or M4 comprises at least one vitamin B6 derivative chosen from the group consisting of pyridoxine, pyridoxal, pyridoxamine or pyridoxal-5'-phosphate or one of the corresponding physiologically compatible salts.

6. Method according to Claim 5, **characterized in that** shortly prior to use, an agent M3 comprising at least one vitamin B6 derivative chosen from the group consisting of pyridoxine, pyridoxal, pyridoxamine or pyridoxal-5'-phosphate or one of the corresponding physiologically compatible salts is added to the agent M2.

7. Method according to one of Claims 5 or 6, **characterized in that** the agent M2 is a preparation according to one of Claims 2 to 4.

## Revendications

1. Utilisation de dérivés de la vitamine B6, choisis parmi le groupe comprenant la pyridoxine, le pyridoxal, la pyridoxamine ou le pyridoxal-5'-phosphate ou un des sels correspondants physiologiquement acceptables, pour réduire la dégradation de fibres kératiniques, en particulier de cheveux humains, lors du traitement de cheveux par oxydation.

2. Préparations pour le traitement de fibres kératiniques par oxydation, en particulier pour la décoloration des cheveux, contenant au moins un agent d'oxydation, **caractérisées en ce qu'**elles contiennent au moins un dérivé de la vitamine B6, choisi parmi le groupe comprenant la pyridoxine, le pyridoxal, la pyridoxamine ou le pyridoxal-5'-phosphate ou un des sels correspondants physiologiquement acceptables.

3. Préparations selon la revendication 2, **caractérisées en ce qu'**elles contiennent les dérivés de la vitamine B6 en une quantité de 0,05 à 2 % en poids, rapportés à la préparation totale.

4. Préparations selon l'une quelconque des revendications 2 ou 3, **caractérisées en ce qu'**elles contiennent en outre un composant faisant office de renforçateur.

5. Procédé pour la décoloration de fibres kératiniques, dans lequel
on applique, si on le souhaite, un agent de prétraitement M1 sur les fibres, puis
- on applique un agent de décoloration M2, contenant au moins un agent d'oxydation, sur les fibres, en ajoutant, si on le souhaite, à l'agent M2, directement avant l'application, un agent supplémentaire M3,
- on élimine des fibres par rinçage cet agent de décoloration M2 après un laps de temps de 5 à 30 minutes
- et après le traitement, on applique éventuellement un agent M4 sur les fibres que l'on élimine à nouveau par rinçage après un temps d'action de quelques minutes,
**caractérisé en ce qu'**au moins un des agents M1, M2, M3 ou M4 contient au moins un dérivé de la vitamine B6, choisi parmi le groupe comprenant la pyridoxine, le pyridoxal, la pyridoxamine ou le pyridoxal-5'-phosphate ou un des sels correspondants physiologiquement acceptables.

6. Procédé selon la revendication 5, **caractérisé en ce qu'**on ajoute à l'agent M2, peu de temps avant l'application, un agent M3 contenant au moins un dérivé de la vitamine B6, choisi parmi le groupe comprenant la pyridoxine, le pyridoxal, la pyridoxamine ou le pyridoxal-5'-phosphate ou un des sels correspondants physiologiquement acceptables.

7. Procédé selon l'une quelconque des revendications 5 ou 6, **caractérisé en ce que** l'agent M2 est une préparation selon l'une quelconque des revendications 2 à 4.
